# EUROPEAN PATENT SPECIFICATION

(11) **EP 0 835 096 B1**
(45) Date of publication and mention of the grant of the patent: **05.09.2001**
(21) Application number: 96917103.2
(22) Date of filing: 04.06.1996
(51) Int. Cl.: A61K 7/50

(54) **LIQUID PERSONAL CLEANSING COMPOSITIONS CONTAINING POLYVALENT METAL CATIONS**
MEHRWERTIGE METALLKATIONEN ENTHALTENDE FLÜSSIGE KÖRPERREINIGUNGSZUSAMMENSETZUNGEN
COMPOSITIONS DE NETTOYAGE LIQUIDES A USAGE PERSONNEL CONTENANT DES CATIONS METALLIQUES POLYVALENTS

(30) Priority: 26.06.1995 US 494478
(43) Date of publication of application: 15.04.1998
(73) Proprietor: THE PROCTER & GAMBLE COMPANY, Cincinnati, Ohio 45202 (US)
(72) Inventor: TUNIS, Adam, Michael, West Chester, OH 45069 (US); GORDON, Gail, Cincinnati, OH 45224 (US); GROSGOGEAT, Eric, Jean, Cincinnati, OH 45208 (US); MacGILP, Neil, Archibald, Sharonville, OH 45241 (US)
(74) Representative: Woof, Victoria
(86) International application number: US9608661
(87) International publication number: WO9701328

(56) References cited:
- EP-A- 0 442 519
- WO-A-92/06669
- WO-A-93/19149
- WO-A-94/17166
- WO-A-94/18292
- WO-A-95/01152
- US-A- 5 227 156

## Description

This invention relates to liquid personal cleansing compositions which contain polyvalent metal cations. The liquid personal cleansing compositions of the present invention additionally comprise an anionic surfactant, an amphoteric surfactant, a skin conditioner ingredient and water. These compositions have a viscosity ranging from about 1,000 to about 50,000 cps. Such compositions are low cost, very mild to the skin, and have an improved rinse profile compared to certain compositions which do not contain polyvalent metal cations.

### BACKGROUND OF THE INVENTION

Liquid personal cleansing products are becoming increasing more popular in the United States and around the world. Desirable liquid personal cleansing compositions must meet a variety of criteria. For example, in order to be acceptable to consumers, a liquid personal cleansing product must exhibit good cleaning properties, mildness/low irritancy with respect to the skin, and good lathering and rinse feel properties.

Although it is well known that ideal personal cleansing products should cleanse the skin gently, without drying or irritating the skin, most lathering soaps, shower and bath products and bars fail in this respect. Certain synthetic surfactants are known to be mild. However, a major drawback of most mild synthetic surfactant systems is that personal cleansing products into which they are incorporated have poor lather performance compared to the highest soap bar standards. Thus, surfactants that are among the mildest, such as sodium lauryl glyceryl esther sulfonate (AGS), contribute to marginal lathering characteristics of the product into which they are incorporated. The use of known high sudsing anionic surfactants with lather boosters, on the other hand, can yield acceptable lather volume and quality, but at the expense of clinical skin mildness. These two facts make the surfactant selection, the lather and mildness benefit formulation process a delicate balancing act.

Despite the many years of research that have been expended by the toiletries industry on personal cleansing, the broad mass of consumers remain dissatisfied with the mildness of present day cleansing compositions, finding, for example, that they have to apply a separate cosmetic lotion or cream moisturizer to the skin after using a shower or bath preparation in order to maintain skin suppleness an dehydration and the counteract the delipidizing effect of the cleanser.

U.S. Patent 5,409,640 to Giret et al; Issued April 25, 1995 and U.S. Patent Application Serial No. 08/327,911 describe certain liquid personal cleansing compositions which exhibit excellent mildness. Moreover, when these liquid personal cleansing compositions are used in combination with the polymeric diamond meshed sponge described in U.S. Patent Application Serial No. 08/327,911, they exhibit excellent lathering characteristics. These liquid personal cleansing compositions comprise certain anionic surfactants, certain amphoteric surfactants, skin conditioner ingredients and water.

U.S. Patent 5,409,640 describes and exemplifies personal cleansing compositions wherein the anionic surfactant consists entirely of sodium laureth-3 sulfate. Unfortunately, it has now been found that personal cleansing products which contain sodium laureth sulfates as the sole anionic surfactant can have a less than optimal rinse profile when they are used with hard water. For example, when these types of compositions were tested in consumer tests under hard water conditions, consumers reported that they rinsed away too easily. These consumers required a slick rinse feel in order to expect good skin effects. If the composition rinsed away too easily and did not leave behind an oily feeling, the consumers perceived this negatively.

It has now been found that the compositions of the present invention, which contain polyvalent metal cations, have comparable mildness and lathering properties to the compositions described in U.S. Patent 5,409,640, but exhibit a superior rinse profile, particularly under hard water conditions, compared to compositions which contain sodium laureth -3 sulfate as the sole anionic surfactant and which do not contain polyvalent metal cations.

### SUMMARY OF THE INVENTION

The present invention relates to liquid personal cleansing compositions which are relatively low cost to prepare, but which exhibit excellent mildness and rinse feel properties. These compositions comprise from 1% to 30% of an anionic surfactant wherein at least 75% of the anionic surfactant comprises sodium laureth-n sulfate, where n ranges from 1 to 12; from 1% to 15% of an amphoteric surfactant, from 0.5% to 5% of a magnesium polyvalent metal cation, water and from 0.1% to 30% of a skin conditioner ingredient comprising an adduct prepared from vegetable oils containing non-conjugated polyunsaturated fatty acid esters which are conjugated and elaidinized and then modified via Diels-Alder addition with a member of the group consisting of acrylic acid, fumaric acid and maleic anhydride.

The weight ratio of the anionic surfactant to the amphoteric surfactant ranges from 4:1 to 1:4. The viscosity of the liquid personal cleansing compositions of the present invention ranges from 1,000 to 50,000 cps.

### DETAILED DESCRIPTION OF THE INVENTION

The present invention relates to liquid personal cleansing compositions which contain magnesium polyvalent metal cations. These polyvalent metal cations, when used in the disclosed amounts in combination with the disclosed amounts of the anionic and amphoteric surfactants described herein, contribute to an improved rinse profile for the personal cleansing compositions herein. The liquid personal cleansing compositions of the present invention exhibit good mildness properties and are relatively inexpensive to manufacture. Moreover, when used in combination with a light weight polymeric diamond meshed sponge, such as that disclosed in U.S. Patent 5,144,744 to Campagnoli; Issued September 8, 1992, the liquid personal cleansing compositions of the present invention exhibit good lathering properties.

The liquid personal cleansing compositions of the present invention are described in detail as follows:

### I. The Ingredients

### A. The Anionic Surfactant

The liquid personal cleansing compositions of the present invention contain from 1% to 30% by weight of the composition of an anionic surfactant. Preferably, the liquid personal cleansing compositions of the present invention contain from 3% to 17% by weight of an anionic surfactant, more preferably from 6% to 14%, and most preferably from 8% to 12%.

Anionic surfactants suitable for inclusion in the compositions of the present invention can generally be described as mild synthetic detergent surfactants. These include, for example, ethoxylated alkyl sulfates, alkyl glyceryl sulfonates, methyl acyl taurates, fatty acyl glycinates, N-acyl glutamates, acyl isethionates, alkyl sulfosuccinates, alpha-sulfonated fatty acids, their salts and/or esters, alkylphosphate esters, ethoxylated alkyl phosphate esters. acyl sarcosinates and fatty acid/protein condensates, and mixtures thereof Alkyl and/or acyl chain lengths for these surfactants are C8-C22, preferably C10-C18.

Preferred for use herein from the standpoint of optimum mildness and lathering/rinse characteristics are the salts of sulfuric acid esters of the reaction product of 1 mole of a higher fatty acid alcohol and from 1 to 12 moles of ethylene oxide, with sodium and magnesium being the preferred counterion. Particularly preferred are the alkyl sulfates containing from 2 to 4 moles of ethylene oxide, such as sodium laureth-2 sulfate, magnesium sodium laureth-3.6 sulfate and sodium laureth-3 sulfate. From a cost standpoint, however, it is desirable to minimize the level of magnesium sodium laureth sulfates present in the liquid personal cleansing compositions herein. Therefore, sodium laureth-n sulfate, wherein n=2-4, is most preferred for use herein.

The anionic surfactant preferably comprises less than 10%, more preferably less than 5%, most preferably less than 1%, magnesium sodium laureth-n sulfate, wherein n=1-12. The anionic surfactant comprises at least about 75%, preferably at least about 90% by weight of sodium laureth-n sulfate, wherein n = 1-12, more preferably wherein n = 2-4.

### B. The Amphoteric Surfactant

The liquid personal cleansing compositions of the present invention contain from 1% to 15% by weight of an amphoteric surfactant. Preferably the liquid personal cleansing compositions of the present invention comprise from 3% to 12%, more preferably from 4% to 8% of an amphoteric surfactant.

Amphoteric surfactants suitable for use in the compositions of the invention include:
(a) imidazolinium surfactants of formula (II) 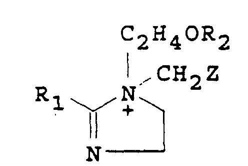 wherein R₁ is C₇-C₂₂ alkyl or alkenyl, R₂ is hydrogen or CH₂Z, each Z is independently CO₂M or CH₂CO₂M, and M is H, alkali metal, alkaline earth metal, ammonium or alkanolammonium; and/or ammonium derivatives of formula (III) 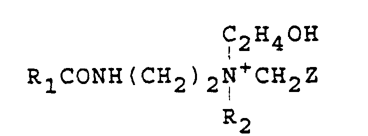 wherein R₁, R₂ and Z are as defined above;
(b) aminoalkanoates of formula (IV)

   R₁NH(CH₂)ₙCO₂M

   and iminoclialkanoates of formula (V)

   R₁N[(CH₂)ₘCO₂M]₂

   wherein n and m are numbers from 1 to 4, and R₁ and M are independently selected from the groups specified above; and
(c) mixtures thereof.

Suitable amphoteric surfactants of type (a) are marketed under the trade name Miranol and are understood to comprise a complex mixture of species. Traditionally, the Miranols have been described as having the general formula II, although the CTFA Cosmetic Ingredient Dictionary, 3rd Edition indicates the non-cyclic structure III. In practice, a complex mixture of cyclic and non-cyclic species is likely to exist and both definitions are given here for sake of completeness. Preferred for use herein, however, are the non-cyclic species.

Examples of suitable amphoteric surfactants of type (a) include compounds of formula II and/or III in which R₁ is C₈H₁₇ (especially iso-capryl), C₉H₁₉ and C₁₁H₂₃ alkyl. Especially preferred are the compounds in which R₁ is C₉H₁₉, Z is CO₂M and R₂ is H; the compounds in which R₁ is C₁₁H₂₃, Z is CO₂M and R₂ is CH₂CO₂M; and the compounds in which R₁ is C₁₁H₂₃, Z is CO₂M and R₂ is H.

In CTFA nomenclature, materials suitable for use in the present invention include sodium lauroamphoacetate, cocoamphocarboxypropionate, cocoamphocarboxy propionic acid, and cocoamphoacetate and cocoamphodiacetate (otherwise referred to as cocoamphocarboxyglycinate). Sodium lauroamphoacetate is preferred for use herein. Specific commercial products include those sold under the trade names of Macham 1L-80 (MacIntyre), Empigen CDL60 and CDR 60 (Albright & Wilson), Miranol C2M Conc. N.P., Miranol C2M Conc. O.P., Miranol C2M SF, Miranol CM Special (Miranol, Inc.); Alkateric 2CIB (Alkaril Chemicals); Amphoterge W-2 (Lonza, Inc.); Monateric CDX-38, Monateric CSH-32 (Mona Industries); Rewoteric Am-2C (Rewo Chemical Group); and Schercotic MS-2 (Scher Chemicals).

It will be understood that a number of commercially-available amphoteric surfactants of this type are manufactured and sold in the form of electroneutral complexes with, for example, hydroxide counterions or with anionic sulfate or sulfonate surfactants, especially those of the sulfated C₈-C₁₈ alcohol, C₈-C₁₈ ethoxylated alcohol or C₈-C₁₈ acyl glyceride types. Preferred from the viewpoint of mildness and product stability, however, are compositions which are essentially free of (non-ethoxylated) sulfated alcohol surfactants. Note also that the concentrations and weight ratios of the amphoteric surfactants are based herein on the uncomplexed forms of the surfactants, any anionic surfactant counterions being considered as part of the overall anionic surfactant component content.

Examples of suitable amphoteric surfactants of type (b) include salts, especially the triethanolammonium salts and salts of N-lauryl-beta-amino propionic acid and N-lauryl-imino-dipropionic acid. Such materials are sold under the trade name Deriphat by General Mills and Mirataine by Miranol Inc. Amphoterics preferred for use herein, however, are those of formula II and/or III.

### C. The Polyvalent Cation

Another essential component of the liquid personal cleansing compositions of the present invention is a magnesium polyvalent metal cation. The polyvalent metal cation component should be present in the liquid personal cleansing composition in soluble, free ion form.

The polyvalent metal cation is preferably added to the liquid personal cleansing compositions herein in the form of a soluble metal salt or metal hydroxide. For example, the polyvalent cation is typically added to the liquid personal cleansing compositions herein in the form of an inorganic salt, such as acetate, halide (e.g., chloride), nitrate, or sulfate. Preferred inorganic salts are chloride, sulfate and acetate. Preferably at least 75%, more preferably at least 90% of the polyvalent metal cations are added to the liquid personal cleansing compositions herein in the form of an inorganic salt or metal hydroxide. The polyvalent cation can also be added in the form of a surfactant, such as an anionic surfactant (including the anionic surfactants hereinbefore described), but the amount of polyvalent cation that is added in the form of a surfactant is preferably minimized. Preferably, less than 10%, more preferably less than 5%, most preferably less than 1% of the salt is in the form of a surfactant.

The liquid personal cleansing compositions of the present invention contain from 0.5% to 5% of magnesium polyvalent metal cations. Preferably, the liquid personal cleansing compositions herein contain from 0.5% to 4.1% polyvalent metal cations, more preferably from 0.5% to 3.1% polyvalent metal cations.

In order to obtain a low cost, mild liquid personal cleansing product of the present invention, which exhibits good rinsing characteristics, it is critical that the level of polyvalent metal cation present in the compositions of the present invention be within the range from 0.5% to 5%. In general, the higher the level of polyvalent cations within this range, the better the rinse feel profile. However, increasing the level of polyvalent cations within this range also has the effect of reducing the lather of the compositions herein. When the level of polyvalent cations in the compositions exceeds about 5%, the lathering characteristics can begin to become unacceptable from a consumer standpoint.

### D. Water

The liquid personal cleansing compositions of the present invention further comprise water. Water is typically present in the liquid personal cleansing compositions herein in an amount ranging from 40% to 65%, preferably from 45% to 60%, more preferably from 50% to 60%. It is preferred to use deionized water when preparing the compositions described herein. Polyvalent metal cations are added to the compositions in salt or base form to increase the free polyvalent cation level in the final composition to within the prescribed range.

Alternatively, tap water or other non-deionized water can be used, and the free polyvalent metal cation level can be increased, as applicable, to within the prescribed ranges hereinbefore described. Alternately, if applicable, the level of free polyvalent metal cations can be decreased by partially deionizing the water or by adding a sequestering agent or chelating agent to the compositions (e.g., ethylene diamine tetraacetic acid).

### E. Skin Conditioner Ingredient

The liquid personal cleansing compositions of the present invention further comprise from 0.1% to 30%, preferably from 0.5% to 25%, more preferably from 2% to 20, most preferably from 5% to 20% a skin conditioner ingredient. The skin conditioner ingredient can comprise any skin conditioner known for use in personal cleansing compositions, such as, for example, the skin conditioner ingredients set forth in CTFA Cosmetic Ingredient Handbook, First Edition (1988), pp. 79-84.

Preferred skin conditioners for use herein include esters of fatty acids; glycerin mono-esters, glycerin di-esters, and glycerin tri-esters; epidermal and sebaceous hydrocarbons; lanolin and derivatives; mineral oil; silicone oil; silicone gum; vegetable oils, vegetable oil adducts, nonionic polymers; cationic polymers, polyols selected from the group consisting of glycerin, glycerol, propylene glycol, polypropylene glycols, polyethylene glycols, ethyl hexanediol, hexylene glycols, and mixtures of any of these ingredients.

The skin conditioner comprising the liquid personal cleansing compositions herein preferably comprises a cationic or nonionic polymer. These polymers are typically present in the liquid personal cleansing compositions at a level from 0.01% to 5%, preferably from 0.04% to 2% and especially from 0.05% to 1%. The polymer is found to be valuable for enhancing the creaminess and quality of the foam as well as providing a skin conditioning utility.

Suitable polymers are high molecular weight materials (mass-average molecular weight determined, for instance, by light scattering, being generally from 2,000 to 3,000,000, preferably from 5,000 to 1,000,000).

Representative classes of polymers include cationic and nonionic polysaccharides; cationic and nonionic homopolymers and copolymers derived from acrylic and/or methacrylic acid; cationic and nonionic cellulose resins; cationic copolymers of dimethyldiallylammonium chloride and acrylic acid; cationic homopolymers of dimethyldiallylammonium chloride; cationic polyalkylene and ethoxypolyalkylene imines; quaternized silicones, and mixtures thereof.

By way of exemplification, cationic polymers suitable for use herein include cationic guar gums such as hydroxypropyl trimethyl ammonium guar gum (d.s. of from 0.11 to 0.22) available commercially under the trade names Jaguar C-14-S(RTM) and Jaguar C-17(RTM) and also Jaguar C-16(RTM), which contains hydroxypropyl substituents (d.s. of from 0.8-1.1) in addition to the above-specified cationic groups, and quaternized cellulose ethers available commercially under the trade names Ucare Polymer JR and Celquat. Other suitable cationic polymers are homopolymers of dimethyldiallylammonium chloride available commercially under the trade name Merquat 100, copolymers of dimethyl aminoethylmethacrylate and acrylamide, copolymers of dimethyldiallylammonium chloride and acrylamide, available commercially under the trade names Merquat 550 and Merquat S, quaternized vinyl pyrrolidone acrylate or methacrylate copolymers of amino alcohol available commercially under the trade name Gafquat, and polyalkyleneimines such as polyethylenimine and ethoxylated polyethylenimine.

The skin conditioner ingredient comprising the liquid personal cleansers herein also preferably comprises a vegetable oil. The vegetable oil preferably comprises from 1% to 15% of the liquid personal cleansing compositions, more preferably from 1% to 10%.

The skin conditioner ingredient comprising the liquid personal cleansers herein also preferably comprise glycerin. Glycerin is typically included at levels of from 1% to 5% of the liquid personal cleansing compositions, preferably from about 2% to $% of the liquid cleansing compositions.

The skin conditioner ingredient comprising the liquid cleansers herein also preferably comprises 1'EG 6 C8/C10 glycerides. PEG 6 is preferably included in the liquid personal cleansing compositions herein at levels ranging from 1% to 5%, more preferably from 2% to 4%.

The skin conditioner ingredient herein comprises a vegetable oil adduct. The compositions of the invention preferably contain from 0.1% to 15%, preferably from 0.25% to 10%, more preferably from 0.5% to 5% of a vegetable oil adduct which preferably has the general formula (I): 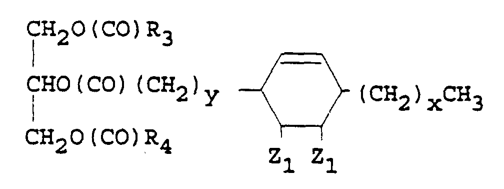 wherein x, y are integers of from 3 to 9, R₃ to R₄ are independently selected from saturated and unsaturated C₇-C₂₂ hydrocarbyl, each Z₁ is CO₂M or H with at least one Z₁ being CO₂M and wherein M is H, alkali metal, alkaline earth metal, ammonium or alkanolammonium.

Materials of this kind can generally be described as adducts prepared from vegetable oils containing non-conjugated polyunsaturated fatty acid esters which are conjugated and elaidinized then modified by Dies-Alder addition with a member of the group consisting of acrylic acid, fumaric acid and maleic anhydride. The adducts and their preparation are described in US-A-4740367, the adducts being marketed under the trade name Ceraphyl GA (Van Dyke).

Preferred vegetable oils adducts are those of Formula I prepared from soybean oil (x + y = 12) and adducts derived by Dies-Alder addition of vegetable oils with fumaric acid. A preferred method of preparing adducts herein is to react two moles of vegetable oil with one mole of the dienophile in the presence of catalytic amounts of iodine, the conjugation and elaidinization agent. This produces a 50:50 blend of adduct together with disproportionated (conjugated) vegetable oil.

### F. Optional Ingredients

### 1. Other Surfactants

The compositions herein preferably also contain from 0.1% to 20%, more preferably from 0.1% to 10%, and especially from 1% to 5% of a nonionic or betaine surfactant. Preferred herein from the viewpoint of optimum lathering and mildness are nonionic surfactants selected from C₁₂-C₁₄ fatty acid mono- and diethanolamides and polyhydroxy fatty acid amine surfactants having the general formula (VII) 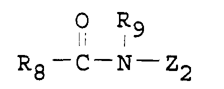 where R₉ is H, C₁-C₄ hydrocarbyl, 2-hydroxyethyl, 2-hydroxypropyl or a mixture thereof, R₈ is C₅-C₃₁ hydrocarbyl and Z₂ is a polyhydroxyhydrocarbyl having a linear chain with at least 3 hydroxyls directly connected to said chain, or an alkoxylated derivative thereof.

The preferred polyhydroxy fatty acid amide surfactants are those in which R₉ is C ₁₋₄ alkyl, preferably methyl, and R₈ is C₇-C₁₉ alkyl or alkenyl, more preferably straight-chain C₉-C₁₇ alkyl or alkenyl, or mixture thereof; and Z₂ is a polyhydroxyhydrocarbyl having a linear hydrocarbyl chain with at least 3 hydroxyls directly connected to the chain, or an alkoxylated derivative (preferably ethoxylated or propoxylated) thereof. Z₂ preferably will be derived from a reducing sugar in a reductive amination reaction; more preferably Z₂ is a glycityl. Suitable reducing sugars include glucose, fructose, maltose, lactose, galactose, mannose, and xylose As raw materials, high dextrose corn syrup, high fructose corn syrup, and high maltose corn syrup can be utilized as well as the individual sugars listed above. These corn syrups may yield a mix of sugar components for Z₂. It should be understood that it is by no means intended to exclude other suitable raw materials. Z₂ preferably will be selected from the group consisting of -CH₂(CHOH)ₙ-CH₂OH,-CH(CH₂OH)-(CHOH)ₙ₋₁-CH₂OH, -CH₂-(CHOH)₂(CHOR')(CHOH)-CH₂OH, where n is an integer from 3 to 5, inclusive, and R' is H or a cyclic or aliphatic monosaccharide, and alkoxylated derivatives thereof. Most preferred are glycityls wherein n is 4, particularly -CH₂-(CHOH)₄-CH₂OH.

The most preferred polyhydroxy fatty acid amide has the formula R₈(CO)N(CH₃)CH₂(CHOH)₄CH₂OH wherein R₈ is a C₁₁-C₁₇ straight chain alkyl or alkenyl group.

Betaine surfactants suitable for inclusion in the composition of the invention include alkyl betaines of the formula R₅R₆R₇N⁺(CH₂)ₙM (VII) and amido betaines of the formula (VIII) 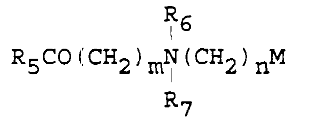 wherein R₅ is C₁₂-C₂₂ alkyl or alkenyl, R₆ and R₇ are independently C₁-C₃ alkyl, M is H, alkali metal, alkaline earth metal, ammonium or alkanolammonium, and n, m are each numbers from 1 to 4. Preferred betaines include cocoamidopropyldimethylcarboxymethyl betaine and laurylamidopropyldimethylcarboxymethyl betaine.

### 2. Other

A number of additional optional materials can be added to the cleansing compositions. Such materials include proteins and polypeptides and derivatives thereof, water-soluble or solubilizable preservatives such as DMDM Hydantoin, Germall 115, methyl, ethyl, propyl and butyl esters of hydroxybenzoic acid, EDTA, Euxyl (RTM) K400, Bronopol (2-bromo-2-nitropropane-1,3-diol), sodium benzoate and 2-phenoxyethanol; other moisturizing agents such as hylaronic acid, chitin , and starch-grafted sodium polyacrylates such as Sanwet (RTM) IM-1000, IM-1500 and IM-2500 available from Celanese Superabsorbent Materials, Portsmith, VA, USA and described in US-A-4,076,663; low temperature phase modifiers such as ammonium ion sources (e.g. NH₄ Cl); viscosity control agents; coloring agents; pearlescers and opacifiers such as ethylene glycol distearate, TiO₂ and TiO₂-coated mica; perfumes and perfume solubilizers etc.

### II. Characteristics of the Liquid Personal Cleansing Compositions

### A. Ratio of Anionic Surfactant to Amphoteric Surfactant

The ratio of the anionic surfactant to amphoteric surfactant in the liquid cleansing compositions of the present invention ranges from 4:1 to 1:4, more preferably from 3:1 to 1:1, most preferably from 2.5:1 to 1.5:1.

### B. Viscosity

The viscosity of the liquid personal cleansing compositions herein ranges from 1,000 to 50,000 cps, preferably from 5,000 to 45,000 cps, most preferably from 10,000 to 40,000 cps. The viscosity can be increased to within the target viscosity range by a variety of means including, but not limited to, the addition of a higher level of polyvalent metal cations within the range hereinbefore described.

The viscosity of the liquid personal cleansing compositions herein can be measured according to the method set forth hereinafter in the Analytical Methods section.

### C. Average Emulsion Droplet Size

The average size of the emulsion droplets within the liquid personal cleansing compositions of the present invention typically ranges from 1 to 20 microns.

### D. pH

The pH of the compositions is preferably from 4 to 9, more preferably from 4.5 to 8.5, most preferably from 4.5 to 7.0, pH being controlled, for example, using a citrate buffer system.

### III. Method of Preparation

A preferred method for preparing the composition herein comprises
a) forming an aqueous phase comprising the water, the anionic and amphoteric surfactants and any water-based skin conditioning ingredients;
b) forming an oil phase comprising the oil-based skin conditioner ingredients and the nonionic surfactants (if they are included in the composition);
c) mixing the oil phase and the aqueous surfactant phase to form an oil-in-water emulsion
d) adding the polyvalent metal cation.

When a vegetable oil adduct is included in the composition, it is added after step (c), but before step (d).

### ANALYTICAL METHODS

### Method for Measuring Viscosity of Liquid Personal Cleansing Compositions Herein

### I. Scope

This method is applicable to the determination of the viscosity of the liquid personal cleanser compositions herein.

### II. Principle

A sample of product is adjusted to 25°C and the viscosity is measured using a cone and plate viscometer.

| III. Apparatus | Suggested Type or Source |
|---|---|
| Viscometer | Brookfield Models RVT DV-II |
| | Viscosity |
| | <12,000 cps >12,000 cps |
| Cone | CP 41 CP-52 |
| RPM | 1.0 |
| Sample | 2 ml |
| Constant Temperature Bath and Flowthru Cooler | Capable of maintaining a temperature of 25°± 0.1°C |
| Thermometer | Accurate; of suitable range; graduated to 0.1°C |
| Syringe | 1 ml or 3 ml for CP 52 measurements; 3 ml for CP 41 measurements |

### IV. Set Up and Calibration

The viscometer should be mechanically set up and calibrated according to the Brookfield Digital Viscometer DV-II Operating Instructions once each day and/or after changing cones.

### V. Checks before Operating

A. Temperature and Bath Cooler. Turn scitches on both the bath and cooler. Make certain flow control on bath is all the way open. Brookfield standards are run at 25°C (very important -1°C deviation can result in a 6-9% difference in viscosity.) Bath temperature should be at least 25°C for standard and for samples.
B. Make sure instrument is level (bubble is centered in circle of level indicator on viscometer).

### VI. Zeroing the Viscometer

A. Remove cone to zero the viscometer. Using wrench, apply to flat portion of spindle; turn spindle, keep wrench stationary.
   - Spindle:: Clockwise is ON
   - Spindle:: Counter-clockwise is OFF
   Note to see if cone is spinning properly. If motion is jerky, send unit back for repair.
B. After cone has been removed, put viscosity cup on, and secure by sliding hinge across bottom of cup.
C. Power on, Motor off.
D. Press Auto zero key.
E. Once screen is finished blinking, replace cone.
F. Turn speed to 1.0 RPM.
G. To select cone, Press Spdl button, and enter cone number.
H. Hit CPS key (This key changes readout from "% of scale" to "cps").
I. Turn motor on, and check movement of cone.
J. Check sample cup for large dings. If these are present, the cup needs to be replaced.

### VII. Setting the Cone to Cup Gap

NOTE: This step will be performed daily, or any time a spindle is changed.
A. Place viscometer cap on instrument.
B. Turn speed knob on left side to 10 RPM.
C. Turn motor on (display will read ---).
D. Press CPS key.
E. Hold sample cup while turning adjustment ring counter-clockwise until a number other than 0.0 is displayed. Let the reading stabilize. Turn the ring clockwise until decrease reading; counter-clockwise will increase the reading.
F. Once the display registers a reading, mark line with a pencil and turn adjustment ring clockwise one deviation (mark).
G. Place transparent tape over ring to keep in place.
H. Turn motor OFF. Set viscometer speed back to 1 RPM calibration.
The viscometer is now ready for measurement.

### VIII. Viscosity Calibration/Measurement

NOTE: Calibration of instrument should be performed 1 time per day minimum, or each time a spindle is changed. When using Brookfield Viscosity Standards, the standard deviation for the viscosity measurement is equal to 1% of full scale of the instrument, PLUS 1% of the rated viscosity of the standard.

### EXAMPLES

The following Examples of illustrative of the liquid personal cleansing compositions of the present invention.

Liquid personal cleansing composition having the following formulations are prepared:

| | **Example I** | **Example II** |
|---|---|---|
| **Component** | | |
| Water | 57.81 | 58.86 |
| Sodium Laureth 3.0 Sulfate | 10.5 | 10 |
| Skin Conditioner Ingredients (Soybean Oil, PEG 6 | 18.7 | 18.8 |
| C8/C10 glycerides, glycerin, palm kernel fatty | | |
| acid, maleated soybean oil, polyquaterium 10) | | |
| Sodium Lauroamphoacetate | 5.25 | 5 |
| Cocamide CMEA | 2.82 | 2.82 |
| Perfume | 1.6 | 1.6 |
| pH adjusters | 1.7 | 1.7 |
| Magnesium sulfate | 1.1 | 0.6 |
| Preservatives | 0.52 | 0.52 |
| Whitener | --- | 0.1 |
| | | |
| | 100 | 100 |
| | | |
| **Physical Properties** | | |
| Viscosity C/P#52, cps | 26,600 | 22,400 |
| % anionic surfactant | 10.32 | 10 |
| % MgSO₄ | 1.1 | 0.6 |

Examples I and II can be prepared according to the following procedure:
1. Clean and Sanitize making equipment.
2. In the main mix tank, form an aqueous phase comprising the water, the polymer (Polyquaternium 10), the amphoteric surfactant (Sodium Lauroamphoacetate), 1/2 of the total anionic surfactant (Sodium Laureth 3.0 Sulfate) and the preservatives and pH adjusters. The ingredients are mixed under agitation to ensure good mixing. The temperature of the mix tank is maintained at from 55-60°C
3. In a separate mix tank, form an oil phase comprising the nonionic surfactant (Monamid CMA) and the oil-based skin conditioner ingredients (PEG-6 Caprylic/Capric Glyceride, palm kernel fatty acids and soybean oil). The temperature of the mix tank is maintained at 55-60°C.
4. Add hot oil phase to main mix tank once both tanks reach temperature.
5. Flush oil phase tank/lines with 0.5% of the formula water
6. Start cooling batch to 25 - 30°C and add the glycerin and the maleated soybean oil
7. Add the remaining half of anionic surfactant, magnesium sulfate and perfume
11. Mix for 30 minutes minimum. Measure product viscosity after 30 minutes.
12. Adjust batch for viscosity, if needed.
13. Add 1% of the total formula water.

The liquid personal cleansing compositions exemplified in Examples I and II are mild to the skin and have an optimal rinse profile. These qualities are perceived by experimental users in terms of positive skin effects.

## Claims

1. A liquid personal cleansing composition **characterized in that** it comprises:
A. from 1% to 30% by weight of an anionic surfactant wherein at least 75% of the anionic surfactant comprises sodium laureth-n sulphate, wherein n ranges from 1 to 12;
B from 1% to 15% by weight of an amphoteric surfactant;
C. from 0.5% to 5% of a magnesium polyvalent metal cation;
D. water; and
E. from 0.1% to 30% of a skin conditioner ingredient comprising an adduct prepared from vegetable oils containing non-conjugated polyunsaturated fatty acid esters which are conjugated and elaidinized and then modified via Diels- Alder addition with a member of the group consisting of acrylic acid, fumaric acid and maleic anhydride;
wherein the weight ratio of the anionic surfactant:amphoteric surfactant ranges from 4:1 to 1:4 and wherein said liquid personal cleansing composition has a viscosity ranging from 1,000 to 50,000 cps.

2. The liquid personal cleansing composition of Claim 1 wherein the anionic surfactant is selected from the group consisting of ethoxylated alkyl sulfates, alkyl glyceryl sulfonates, methyl acyl taurates, fatty acyl glycinates, N-acyl glutamates, acyl. isethionates, alkyl sulfosuccinates, alpha-sulfonated fatty acids, their salts and/or their esters, alkyl phosphate esters, ethoxylated alkyl phosphate esters, acyl sarcosinates and fatty acid/protein condensates, and mixtures thereof.

3. The liquid personal cleansing composition of Claim 2 wherein less than 5% of the anionic surfactant comprises magnesium sodium laureth-n sulfate, wherein n=1-12.

4. The liquid personal cleansing composition of Claim 3 wherein at least 90% of the anionic surfactant comprises sodium laureth-n sulfate, wherein n ranges from 1 to 12.

5. The liquid personal cleansing composition of Claim 4 which additionally comprises from 0.1% to 20% of a nonionic surfactant.

6. The liquid personal cleansing composition of Claim which comprises from 0.5% to 30% of a skin conditioner ingredient wherein the skin conditioner ingredient additionally comprises an ingredient selected from the group consisting of esters of fatty acids; glycerin mono-esters, glycerin di-esters, and glycerin tri-esters; epidermal and sebaceous hydrocarbons; lanolin and derivatives; mineral oil; silicone oil; silicone gum; vegetable oils, additional vegetable oil adducts, nonionic polymers; cationic polymers, polyols selected from the group consisting of glycerin, glycerol, propylene glycol, polypropylene glycols, polyethylene glycols, ethyl hexanediol, hexylene glycols, and mixtures of any of these ingredients.

7. The liquid personal cleansing composition of Claim 6 wherein the amphoteric surfactant is selected from the group consisting of
(a) imidazolinium surfactants of formula (II) 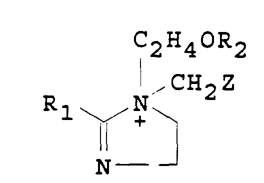 wherein R₁ is C₇-C₂₂ alkyl or alkenyl, R₂ is hydrogen or CH₂Z, each Z is independently CO₂M or CH₂CO₂M, and M is H, alkali metal, alkaline earth metal, ammonium or alkanolammonium; and/or ammonium derivatives of formula (III) 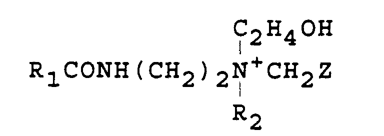 wherein R₁, R₂ and Z are as defined above;
(b) aminoalkanoates of formula (IV)
R₁NH(CH₂)ₙCO₂M
and iminodialkanoates of formula (V)
R₁N[(CH₂)ₘCO₂M]₂
wherein n and m are numbers from 1 to 4, and R₁ and M are independently selected from the groups specified above; and
(c) mixtures thereof.

8. The liquid personal cleansing composition of Claim 7 wherein at least 75% of the polyvalent metal cations are added to the liquid personal cleansing compositions in the form of an inorganic salt.

9. The liquid personal cleansing compositions of Claim 8 wherein the inorganic salt is magnesium sulfate.

10. A liquid personal cleansing composition **characterized in that** it comprises:
A. from 3% to 17% by weight of an anionic surfactant; wherein at least 75% of the anionic surfactant comprises sodium laureth-n sulfate, wherein n ranges from 1 to 12;
B from 3 to 12% by weight of an amphoteric surfactant selected from the group consisting of
(a) imidazolinium surfactants of formula (II) 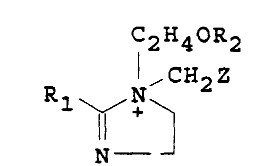 wherein R₁ is C₇-C₂₂ alkyl or alkenyl, R₂ is hydrogen or CH₂Z, each Z is independently CO₂M or CH₂CO₂M, and M is H, alkali metal, alkaline earth metal, ammonium or alkanolammonium; and/or ammonium derivatives of formula (III) 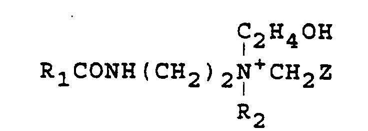 wherein R₁, R₂ and Z are as defined above;
(b) aminoalkanoates of formula (IV)
R₁NH(CH₂)ₙCO₂M
and iminodialkanoates of formula (V)
R₁N[(CH₂)ₘCO₂M]₂
wherein n and m are numbers from 1 to 4, and R₁ and M are independently selected from the groups specified above; and
(c) mixtures thereof;
C. from 0.25% to 10% by weight of an adduct prepared from vegetable oils containing non-conjugated polyunsaturated fatty acid esters which are conjugated and elaidinized and then modified via Diels-Alder addition with a member of the group consisting of acrylic acid, fumaric acid and maleic anhydride;
D. from 0.5 to 4.1% of a magnesium polyvalent metal cation, wherein less than 10% of the polyvalent cation is added to said composition in the form of a surfactant;
E. from 50 to 60% water;
F. from 0.1 to 10% of a nonionic surfactant selected from the group consisting of C₁₂-C₁₄ fatty acid mono- and diethanolamides and polyhydroxy fatty acid amine surfactants having the general formula (VII) 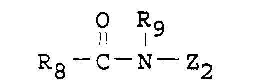 where R₉ is H, C₁-C₄ hydrocarbyl, 2-hydroxyethyl, 2-hydroxypropyl or a mixture thereof, R₈ is C₅-C₃₁ hydrocarbyl and Z₂ is a polyhydroxyhydrocarbyl having a linear chain with at least 3 hydroxyls directly connected to said chain, or an alkoxylated derivative thereof;
G. from 0.04% to 2% of a cationic polymer; and
H. from 1% to 15% of a vegetable oil; and
wherein the weight ratio of the anionic surfactant: amphoteric surfactant ranges from 3:1 to 1:1, wherein said liquid personal cleansing composition has a viscosity ranging from 10,000 to 40,000 cps, and wherein said composition has a pH ranging from 4.5 to 8.5.

## Patentansprüche

1. Flüssige Körperreinigungszusammensetzung, **dadurch gekennzeichnet**, daß sie:
A. 1 Gew.-% bis 30 Gew.-% eines anionischen grenzflächenaktiven Mittels, wobei mindestens 75% des anionischen grenzflächenaktiven Mittels Natriumlaureth-n-sulfat umfassen, worin n von 1 bis 12 reicht;
B. 1 Gew.-% bis 15 Gew.-% von einem amphoteren grenzflächenaktiven Mittel;
C. 0,5% bis 5% von einem mehrwertigen Magnesiummetallkation;
D. Wasser; und
E. 0,1% bis 30% von einem Hautkonditionierungsbestandteil, umfassend ein aus pflanzlichen Ölen hergestelltes Addukt, welche pflanzlichen Öle nicht-konjugierte, mehrfach ungesättigte Fettsäureester enthalten, die konjugiert und elaidinisiert und anschließend durch Diels-Alder-Addition mit einem Mitglied der aus Acrylsäure, Fumarsäure und Maleinsäureanhydrid bestehenden Gruppe modifiziert werden;
umfaßt, wobei das Gewichtsverhältnis vom anionischen grenzflächenaktiven Mittel zum amphoteren grenzflächenaktiven Mittel von 4:1 bis 1:4 reicht und wobei die genannte flüssige Körperreinigungszusammensetzung eine von 1.000 cPs bis 50.000 cPs reichende Viskosität besitzt.

2. Flüssige Körperreinigungszusammensetzung nach Anspruch 1, worin das anionische grenzflächenaktive Mittel von der Gruppe ausgewählt ist, welche aus ethoxylierten Alkylsulfaten, Alkylglycerylsulfonaten, Methylacyltauraten, Fettacylglycinaten, N-Acylglutamaten, Acylisethionaten, Alkylsulfosuccinaten, alpha-sulfonierten Fettsäuren, deren Salzen und/oder deren Estern, Alkylphosphatestern, ethoxylierten Alkylphosphatestern, Acylsarcosinaten und Fettsäure/Protein-Kondensaten und Gemischen hievon besteht.

3. Flüssige Körperreinigungszusammensetzung nach Anspruch 2, worin weniger als 5% des anionischen grenzflächenaktiven Mittels Magnesiumnatriumlaureth-n-sulfat umfassen, worin n=1-12 bedeutet.

4. Flüssige Körperreinigungszusammensetzung nach Anspruch 3, worin mindestens 90% des anionischen grenzflächenaktiven Mittels Natriumlaureth-n-sulfat umfassen, worin n von 1 bis 12 reicht.

5. Flüssige Körperreinigungszusammensetzung nach Anspruch 4, welche zusätzlich 0,1% bis 20% von einem nichtionischen grenzflächenaktiven Mittel umfaßt.

6. Flüssige Körperreinigungszusammensetzung nach Anspruch 5, welche 0,5% bis 30% von einem Hautkonditionierungsbestandteil umfaßt, wobei der Hautkonditionierungsbestandteil zusätzlich einen Bestandteil umfaßt, welcher von der Gruppe ausgewählt ist, die aus Fettsäureestern; Glycerinmonoestern, Glycerindiestern und Glycerintriestern, Epidermal- und Talgkohlenwasserstoffen; Lanolin und Derivaten, Mineralöl, Silikonöl, Silikonkautschuk, pflanzlichen Ölen, zusätzlichen pflanzlichen Öl-Addukten, nichtionischen Polymeren, kationischen Polymeren, Polyolen, welche von der aus Glycerin, Glycerol, Propylenglycol, Polypropylenglycolen, Polyethylenglycolen, Ethylhexandiol, Hexylenglycolen bestehenden Gruppe ausgewählt sind, und Gemischen von jedweden dieser Bestandteile besteht.

7. Flüssige Körperreinigungszusammensetzung nach Anspruch 6, worin das amphotere grenzflächenaktive Mittel von der Gruppe bestehend aus
(a) Imidazolinium-grenzflächenaktiven Mitteln der Formel (II) 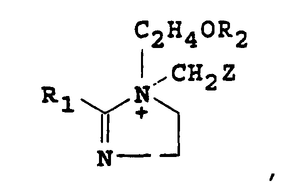 worin R₁ für C₇-C₂₂-Alkyl oder -Alkenyl steht, R₂ Wasserstoff oder CH₂Z darstellt, jeder Rest Z unabhängig CO₂M oder CH₂CO₂M bedeutet und M für H, Alkalimetall, Erdalkalimetall, Ammonium oder Alkanolammonium steht; und/oder Ammoniumderivaten der Formel (III) 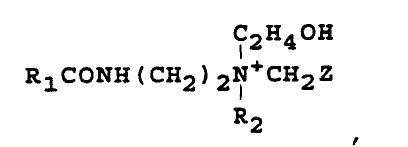 worin R₁, R₂ und Z wie vorstehend definiert sind;
(b) Aminoalkanoaten der Formel (IV)
R₁NH(CH₂)ₙCO₂M
und Iminodialkanoaten der Formel(V)
R₁N[(CH₂)ₘCO₂M]₂,
worin n und m Zahlen von 1 bis 4 sind, und R₁ und M unabhängig von den vorstehend angegebenen Gruppen ausgewählt sind; und
(c) Gemischen hievon ausgewählt ist.

8. Flüssige Körperreinigungszusammensetzung nach Anspruch 7, worin mindestens 75% der mehrwertigen Metallkationen den flüssigen Körperreinigungszusammensetzungen in der Form eines anorganischen Salzes zugesetzt werden.

9. Flüssige Körperreinigungszusammensetzungen nach Anspruch 8, worin das anorganische Salz Magnesiumsulfat ist.

10. Flüssige Körperreinigungszusammensetzung, **dadurch gekennzeichnet**, daß sie
A. 3 Gew.-% bis 17 Gew.-% von einem anionischen grenzflächenaktiven Mittel; wobei mindestens 75% des anionischen grenzflächenaktiven Mittels Natriumlaureth-n-sulfat umfassen, worin n von 1 bis 12 reicht;
B. 3 Gew.-% bis 12 Gew.-% von einem amphoteren grenzflächenaktiven Mittel, welches von der Gruppe bestehend aus:
(a) Imidazolinium-grenzflächenaktiven Mitteln der Formel (II) 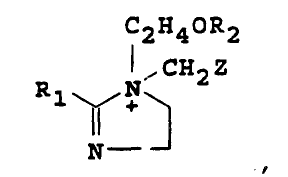 worin R₁ für C₇-C₂₂-Alkyl oder -Alkenyl steht, R₂ Wasserstoff oder CH₂Z darstellt, jeder Rest Z unabhängig CO₂M oder CH₂CO₂M bedeutet und M für H, Alkalimetall, Erdalkalimetall, Ammonium oder Alkanolammonium steht; und/oder Ammoniumderivaten der Formel (III) 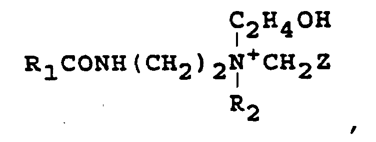 worin R₁, R₂ und Z wie vorstehend definiert sind;
(b) Aminoalkanoaten der Formel (IV)
R₁NH(CH₂)ₙCO₂M
und Iminodialkanoaten der Formel(V)
R₁N[(CH₂)ₘCO₂M]₂,
worin n und m Zahlen von 1 bis 4 sind, und R₁ und M unabhängig von den vorstehend angegebenen Gruppen ausgewählt sind; und
(c) Gemischen hievon, ausgewählt ist;
C. 0,25 Gew.-% bis 10 Gew.-% von einem aus pflanzlichen Ölen hergestellten Addukt, welche pflanzlichen Öle nicht-konjugierte, mehrfach ungesättigte Fettsäureester enthalten, die konjugiert und elaidinisiert und anschließend durch Diels-Alder-Addition mit einem Mitglied der aus Acrylsäure, Fumarsäure und MaleinSäureanhydrid bestehenden Gruppe modifiziert werden;
D. 0,5% bis 4,12% von einem mehrwertigen Magnesiummetallkation, wobei weniger als 10% des mehrwertigen Kations der genannten Zusammensetzung in der Form eines grenzflächenaktiven Mittels zugesetzt werden;
E. 50% bis 60% Wasser;
F. 0,1% bis 10% von einem nichtionischen grenzflächenaktiven Mittel, welches von der Gruppe ausgewählt ist, die aus C₁₂-C₁₄-Fettsäuremono- und -diethanolamiden und Polyhydroxyfettsäureamin-grenzflächenaktiven Mitteln der allgemeinen Formel (VII) 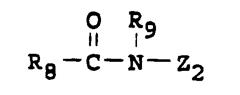 besteht, worin R₉ für H, C₁-C₄-Hydrocarbyl, 2-Hydroxyethyl, 2-Hydroxypropyl oder ein Gemisch hievon steht, R₈ C₅-C₃₁-Hydrocarbyl bedeutet und Z₂ ein Polyhydroxyhydrocarbyl mit einer linearen Kette mit mindestens drei direkt an die genannte Kette gebundenen Hydroxylgruppen ist, oder ein alkoxyliertes Derivat hievon darstellt;
G. 0,04% bis 2% von einem kationischen Polymer; und
H. 1% bis 15% von einem pflanzlichen Öl; umfaßt, und
worin das Gewichtsverhältnis vom anionischen grenzflächenaktiven Mittel zum amphoteren grenzflächenaktiven Mittel von 3:1 bis 1:1 reicht, wobei die genannte flüssige Körperreinigungszusammensetzung eine von 10.000 cPs bis 40.000 cPs reichende Viskosität besitzt und wobei die genannte Zusammensetzung einen pH-Wert von 4,5 bis 8,5 aufweist.

## Revendications

1. Composition de nettoyage liquide pour l'hygiène personnelle
**caractérisée en ce qu**'elle comprend:
A. 1% à 30% en poids d'un agent tensioactif anionique, au moins 75% en poids de l'agent tensioactif anionique étant constitué de laureth-n-sulfate de sodium, où n varie de 1 à 12;
B. 1% à 15% en poids d'un agent tensioactif amphotère;
C. 0,5% à 5% en poids d'un cation métallique polyvalent de magnésium;
D. de l'eau; et
E. 0,1% à 30% d'un ingrédient de conditionnement de la peau, comprenant un produit d'addition préparé à partir d'huiles végétales contenant des esters d'acides gras polyinsaturés non conjugués qui sont conjugués et élaïdinisés, puis modifiés par une addition de Diels-Alder avec un membre du groupe constitué par l'acide acrylique, l'acide fumarique et l'anhydride maléique;
le rapport pondéral de l'agent tensioactif anionique à l'agent tensioactif amphotère s'échelonnant de 4:1 à 1:4, et ladite composition de nettoyage liquide pour l'hygiène personnelle ayant une viscosité allant de 1000 à 5000 cP.

2. Composition de nettoyage liquide pour l'hygiène personnelle de la revendication 1 dans laquelle l'agent tensioactif anionique est choisi dans le groupe constitué par les alkylsulfates éthoxylés, les alkylglycérylsulfonates, les méthylacyltaurates, les acylglycinates gras, les N-acylglutamates, les acyliséthionates, les alkylsulfosuccinates, les acides gras alpha-sulfonés, leurs sels et/ou leurs esters, les esters d'alkylphosphate, les esters d'alkylphosphate éthoxylés, les acylsarcosinates et les produits de condensation acides gras/protéines, et leurs mélanges.

3. Composition de nettoyage liquide pour l'hygiène personnelle de la revendication 2 dans laquelle moins de 5% de l'agent tensioactif anionique est constitué de laureth-n-sulfate de sodium, où n = 1 à 12.

4. Composition de nettoyage liquide pour l'hygiène personnelle de la revendication 3 dans laquelle au moins 90% de l'agent tensioactif anionique est constitué de laureth-n-sulfate de sodium, où n va de 1 à 12.

5. Composition de nettoyage liquide pour l'hygiène personnelle de la revendication 4, qui comprend en outre de 0,1 % à 20 % d'un agent tensioactif non ionique.

6. Composition de nettoyage liquide pour l'hygiène personnelle de la revendication 5, qui comprend de 0,5 % à 30 % d'un ingrédient de conditionnement de la peau, où l'ingrédient de conditionnement de la peau comprend en outre un ingrédient du groupe constitué par les esters d'acides gras; les monoesters de glycérine, les diesters de glycérine et les triesters de glycérine; les hydrocarbures épidermiques et sébacés; la lanoline et ses dérivés; l'huile minérale; l'huile de silicone; la gomme de silicone; des huiles végétales, des produits d'addition d'huiles végétales additionnels, des polymères non ioniques; des polymères cationiques, des polyols choisis dans le groupe constitué par la glycérine, le glycérol, le propylèneglycol, les polypropylèneglycols, les polyéthylèneglycols, l'éthylhexanediol, les hexylèneglycols, et des mélanges quelconques de ces ingrédients.

7. Composition de nettoyage liquide pour l'hygiène personnelle de la revendication 6 dans laquelle l'agent tensioactif amphotère est choisi dans le groupe constitué par
(a) les agents tensioactifs de type imidazolinium de formule (II) 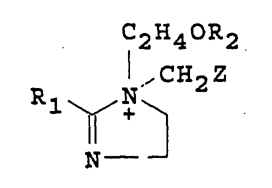 dans laquelle R₁ est un alkyle ou un alcényle en C₇ à C₂₂, R₂ est un hydrogène ou CH₂Z, chaque Z représente indépendamment CO₂M ou CH₂CO₂M, et M représente H, un métal alcalin, un métal alcalino-terreux, l'ammonium ou un alcanol ammonium; et/ou les dérivés d'ammonium de formule (III) 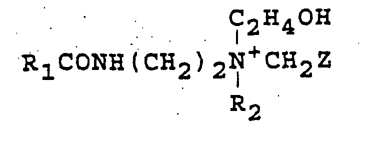 dans laquelle R₁, R₂ et Z sont tels que définis ci-dessus;
(b) les aminoalcanoates de formule (IV)
R₁NH(CH₂)ₙCO₂M
et les iminodialcanoates de formule (V)
R₁N[(CH₂)ₘCO₂M]₂
formules dans lesquelles n et m sont des nombres allant de 1 à 4, et R₁ et M sont choisis indépendamment dans les groupes spécifiés ci-dessus; et
(c) leurs mélanges.

8. Composition de nettoyage liquide pour l'hygiène personnelle de la revendication 7 dans laquelle au moins 75 % des cations métalliques polyvalents sont ajoutés aux compositions de nettoyage liquide pour l'hygiène personnelle sous la forme d'un sel inorganique.

9. Compositions de nettoyage liquide pour l'hygiène personnelle de la revendication 8 dans lesquelles le sel inorganique est le sulfate de magnésium.

10. Composition de nettoyage liquide à usage personnel,
**caractérisée en ce qu**'elle comprend
A. 3 % à 17 % en poids d'un agent tensioactif anionique; où au moins 75 % de l'agent tensioactif anionique est constitué de laureth-n-sulfate de sodium, n allant de 1 à 12;
B. 3 à 12 % en poids d'un agent tensioactif amphotère choisi dans le groupe constitué par
(a) les agents tensioactifs de type imidazolinium de formule (II) 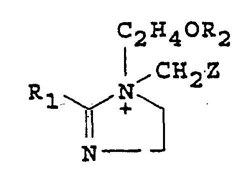 dans laduelle R₁ est un alkyle ou un alcényle en C₇ à C₂₂, R₂ est un hydrogène ou CH₂Z, chaque Z représente indépendamment CO₂M ou CH₂CO₂M, et M représente H, un métal alcalin, un métal alcalino-terreux, l'ammonium ou un alcanolammonium; et/ou les dérivés d'ammonium de formule (III) 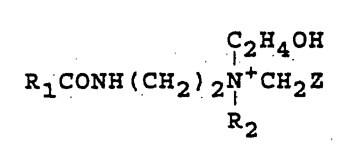 dans laquelle R₁, R₂ et Z sont tels que définis ci-dessus;
(b) les aminoalcanoates de formule (IV)
R₁NH(CH₂)ₙCO₂M
et les iminodialcanoates de formule (V)
R₁N[(CH₂)ₘCO₂M]₂
formules dans lesquelles n et m sont des nombres allant de 1 à 4, et R₁ et M sont choisis indépendamment dans les groupes spécifiés ci-dessus; et
(c) leurs mélanges;
C. 0,25 % à 10 % en poids d'un produit d'addition préparé à partir d'huiles végétales contenant des esters d'acides gras polyinsaturés non conjugués qui sont conjugués et élaïdinisés puis modifiés par une addition de Diels-Alder avec un membre du groupe constitué par l'acide acrylique, l'acide fumarique et l'anhydride maléique;
D. 0,5 à 4,1 % d'un cation métallique polyvalent de magnésium, où moins de 10 % du cation polyvalent est ajouté à ladite composition sous la forme d'un agent tensioactif;
E. 50 à 60 % d'eau;
F. 0,1 à 10 % d'un agent tensioactif non ionique choisi dans le groupe constitué par les mono- et di-éthanolamides d'acides gras en C₁₂ à C₁₄ et les agents tensioactifs de type amine d'acide gras polyhydroxylé de formule générale (VII) 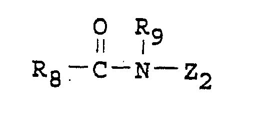 dans laquelle R₉ représente H, un hydrocarbyle en C₁ à C₄, un 2-hydroxyéthyle, un 2-hydroxypropyle ou un de leurs mélanges, R₈ est un hydrocarbyle en C₅ à C₃₁ et Z₂ est un polyhydroxyhydrocarbyle ayant une chaîne linéaire avec au moins trois hydroxyle reliés directement à ladite chaîne, ou un de leurs dérivés alcoxylés;
G. 0,04 % à 2 % d'un polymère cationique, et
H. 1 % à 15 % d'une huile végétale; et
où le rapport pondéral de l'agent tensioactif anionique à l'agent tensioactif amphotère va de 3:1 à 1:1, ladite composition de nettoyage liquide pout l'hygiène personnelle ayant une viscosité allant de 10 000 à 40 000 cP, et ladite composition ayant un pH allant de 4,5 à 8,5.
